# EUROPÄISCHE PATENTSCHRIFT

(11) **EP 0 763 124 B1**
(45) Veröffentlichungstag und Bekanntmachung des Hinweises auf die Patenterteilung: **28.01.2004**
(21) Anmeldenummer: 95920875.2
(22) Anmeldetag: 24.05.1995
(51) Int. Cl.: C12N 15/87, C12N 5/10, A61K 47/48, A61K 48/00

(54) **VERFAHREN ZUM EINBRINGEN VON NUKLEINSÄURE IN HÖHERE EUKARYOTISCHE ZELLEN**
METHOD FOR INTRODUCING NUCLEIC ACID IN HIGHER EUKARYOTIC CELLS
PROCEDE D'INTRODUCTION D'ACIDE NUCLEIQUE DANS DES CELLULES EUCARYOTES D'ORDRE SUPERIEUR

(30) Priorität: 31.05.1994 DE 4418965
(43) Veröffentlichungstag der Anmeldung: 19.03.1997
(73) Patentinhaber: Boehringer Ingelheim International GmbH, 55218 Ingelheim am Rhein (DE)
(72) Erfinder: BUSCHLE, Michael, A-2345 Brunn am Gebirge (AT); WAGNER, Ernst, A-2103 Langenzersdorf (AT); ZAUNER, Wolfgang, A-1030 Wien (AT)
(86) Internationale Anmeldenummer: PCT/EP1995/001969
(87) Internationale Veröffentlichungsnummer: WO 1995/033061

(56) Entgegenhaltungen:
- EP-A- 0 257 993
- EP-A- 0 424 688
- WO-A-91/00915
- WO-A-92/05262
- WO-A-93/19768
- US-A- 4 608 339
- US-A- 4 652 522
- PROC.NAT.ACAD.SCI.USA, Bd. 87,Nr. 4, 1990 Seiten 4033-4037, M.COTTEN ET AL. 'Transferrin-polycation-mediated introduction of DNA into human leukemic cells :stimulation by agents that affect the survival of transfected DNA or modulate transferrin receptor levels'
- BIOCHIM BIOPHYS ACTA, 815 (3). 1985. 515-518., ARNOLD K ET AL 'THE DIELECTRIC PROPERTIES OF AQUEOUS SOLUTIONS OF POLYETHYLENEGLYCOL AND THEIR INFLUENCE ON MEMBRANE STRUCTURE'
- J INVEST DERMATOL, MAY 1994, 102 (5) P768-72, UNITED STATES, STAEDEL C ET AL 'High-efficiency transfection of primary human keratinocytes with positively charged lipopolyamine:DNA complexes.'
- DATABASE WPI Section Ch, Week 8824 Derwent Publications Ltd., London, GB; Class B04, AN 88-164720 & JP,A,63 102 682 ( MEDISA SHINYAKU KK) , 7.Mai 1988

## Beschreibung

Die vorliegende Erfindung bezieht sich auf ein Verfahren zum Einbringen von Nukleinsäure in höhere eukaryotische Zellen.

Bedarf an einem effizienten System für das Einführen von Nukleinsäure in lebende Zellen besteht vor allem im Rahmen der Gentherapie. Dabei werden Gene in Zellen eingeschleust, um in vivo die Synthese therapeutisch wirksamer Genprodukte zu erzielen.

Standardtransfektionsmethoden verwenden u.a. Calciumphosphat, kationische Lipide oder Liposomen, wobei zwecks Verbesserung der Transfektionseffizienz für einige dieser Methoden eine osmotische Schockbehandlung der Zellen mit Glycerin oder Dimethylsulfoxid vorgeschlagen wurde (Chen und Okayama, 1988; Parker und Stark, 1979; Okada und Rechsteiner, 1982).

Die bisher am weitesten fortgeschrittenen Technologien für die Anwendung von Nukleinsäuren im Rahmen der Gentherapie benutzen retrovirale Systeme für den Transfer von Genen in die Zelle (Wilson et al., 1990; Kasid et al., 1990).

Alternativen Strategien für den Gentransfer liegen Mechanismen zugrunde, deren sich die Zelle für den Transport von Makromolekülen bedient. Ein Beispiel dafür ist der Import von Genen in die Zelle über den Weg der Rezeptor-vermittelten Endozytose (z.B. Wu und Wu, 1987, Wagner et al., 1990, und EP-A1 0388 758).

Für den Gentransfer mit DNA/Polykation-Komplexen mittels Rezeptor-vermittelter Endozytose wurde eine Verbesserung vorgeschlagen, die den Einsatz von Komponenten aufgrund ihrer Fähigkeit vorsieht, den Inhalt von Endosomen freisetzen zu können, z.B. Adenoviren oder fusogene Peptide. Der Einsatz der endosomolytischen Komponenten bewirkt eine Steigerung der Effizienz des Gentransfers, indem der Abbau der in die Zelle internalisierten DNA-Komplexe in den Lysosomen vermieden wird (Curiel et al., 1991; Curiel et al., 1992a; Zatloukal et al., 1992; Cotten et al., 1992; Wagner et al., 1992; Curiel et al., 1992b; WO 93/07283). U.a. wurde vorgeschlagen, die Adenoviren durch Bindung an Polylysin zu modifizieren. Die Adenovirus-Polylysin-Konjugate können zusammen mit Konjugaten aus Transferrin-Polylysin mit DNA komplexiert werden, wobei ternäre Transferrin-Polylysin/Adenovirus-Polylysin/DNA-Komplexe entstehen (Wagner et al., 1992). Die Komplexe binden an Transferrin- und Adenovirusrezeptoren auf den Zielzellen. Nach der Endozytose bewirkt das Adenovirus den Aufbruch von Endosomen, das hat die Freisetzung des Materials vom Endosom ins Zytoplasma zur Folge. Diese Technik weist gegenüber konventionellen viralen Techniken eine erhöhte Sicherheit auf (Cotten et al., 1992).

Der vorliegenden Erfindung lag die Aufgabe zugrunde, ein Verfahren zum Einbringen von mit Polykationen komplexierter Nukleinsäure in die Zelle bereitzustellen, das eine weitere Verbesserung hinsichtlich Einfachheit der Durchführung sowie Sicherheit darstellt.

Die Aufgabe wurde gelöst durch ein Verfahren zum Einbringen von Nukleinsäure/Polykation-Komplexen in höhere eukaryotische Zellen, bei dem man die Komplexe in Gegenwart von Ethylenglycol und/oder oder Glycerin auf die Zellen aufbringt.

Im Unterschied zu den für die Calciumphosphat-Präzipitations- oder die DEAE-Dextran-Methode vorgeschlagenen Behandlungen mit Glycerin oder Dimethylsulfoxid, die als Nachbehandlung im Anschluß an die Aufbringung der Transfektionskomponenten vorgenommen werden, womit der beabsichtigte osmotische Schock hervorgerufen wird, ist beim erfindungsgemäßen Verfahren der mehrwertige Alkohol während der ganzen Zeit der Transfektion im Medium. Der mehrwertige Alkohol liegt zweckmäßig als Bestandteil des Transfektionsmediums vor. Aufgrund der Ergebnisse der im Rahmen der vorliegenden Erfindung durchgeführten Experimente kann angenommen werden, daß der mehrwertige Alkohol keine osmotische Wirkung hat, sondern einen anderen Mechanismus auslöst.

Die optimale Konzentration von Glycerin und/oder Ethylenglycol hängt von verschiedenen Faktoren, insbesondere vom Zelltyp ab; sie kann mittels Titrationsversuchen ermittelt werden. Im Rahmen der vorliegenden Erfindung hat sich als Ergebnis solcher Titrationen eine Konzentration von ca. 8 bis ca. 15 % (v/v), insbesondere ca. 8 bis ca. 13 %, bezogen auf das Transfektionsmedium, als günstig erwiesen.

Zusätzlich zu Glycerin und/oder Ethylenglycol können dem Medium Substanzen zugesetzt werden, die die Ansäuerung der Endosomen und somit deren Umwandlung zu sekundären Lysosomen verhindern (Seglen, 1983). Dazu zählen die auch als "lysosomatrope Substanzen" bezeichneten Verbindungen. Vertreter dieser Gruppe von Verbindungen sind Chloroquin, Monensin, Nigericin, Ammoniumchlorid und Methylamin.

Bevorzugt wird im Rahmen der vorliegenden Erfindung Chloroquin in einer Konzentration von ca. 10 bis ca. 300 µM, insbesondere 100 µM, eingesetzt.

Ein weiterer bevorzugter Vertreter dieser Substanzgruppe ist Bafilomycin A1, ein spezifischer Inhibitor der vakuolären Protonenpumpe (Bowman et al., 1988; Yoshimori et al., 1991), das in Konzentrationen von ca. 10 nM bis ca. 1 µM, insbesondere ca. 200 nM eingesetzt wird.

Die Auswahl dieser Substanzen und deren Konzentration richtet sich nach dem zu behandelnden Zelltyp, diese Parameter können in Vorversuchen bestimmt werden, in denen untersucht wird, ob die jeweiligen Substanzen in verschiedenen Konzentrationen eine Expressionssteigerung bewirken können oder toxisch sind.

Eine weitere Substanzgruppe, die die Wirkung von Glycerin und/oder Ethylenglycol zu steigern vermag, sind niedere einwertige Alkohole, insbesondere Ethanol.

Ethanol wird in nicht-toxischen Konzentrationen (etwa bis zu 3 %), insbesondere in einer Konzentration von 1 bis 1.5 % zugesetzt.

Nukleinsäure/Polykation-Komplexe sind Stand der Technik; hinsichtlich der Zusammensetzung, der qualitativen und quantitativen Anforderungen an die Komplexkomponenten sowie hinsichtlich der Herstellung der Komplexe wird auf die WO 93/07283 verwiesen: Die DNA- und RNA-Moleküle, insbesondere therapeutisch wirksame Nukleinsäure-Moleküle, werden durch den Anwendungszweck definiert, es bestehen keine Beschränkungen hinsichtlich ihrer Sequenz; hinsichtlich der Größe der Moleküle ist die Verwendbarkeit für einen weiten Bereich gegeben. Geeignete Polykationen sind u.a. homologe organische Polykationen wie Polylysin, Polyarginin, Polyornithin oder heterologe Polykationen mit zwei oder mehr unterschiedlichen positiv geladenen Aminosäuren, wobei diese Polykationen verschiedene Kettenlänge aufweisen können, weiters nicht-peptidische synthetische Polykationen wie Polyethylenimin. Als Polykationen können auch sphäroide Polykationen, die als "Starburst Dendrimere" bekannt geworden sind (Haensler und Szoka, 1993) eingesetzt werden. Geeignet sind ferner natürliche DNA-bindende Proteine polykationischen Charakters wie Histone oder Protamine bzw. Analoge oder Fragmente davon, sowie Spermin oder Spermidine. Gegebenenfalls sind die Polykationen modifiziert, z.B. durch lipophile Gruppierungen wie Kohlenwasserstoffgruppierungen mit Ähnlichkeit zu natürlichen Lipiden (z.B. Fettsäuren, Cholesterin), wodurch die die Affinität der Komplexe zur Zellmembran erhöht wird. Eine weitere Möglichkeit zur Modifikation besteht in hydrophilen Gruppierungen, die geeignet sind, die Spezifität der Komplexe für die Zielzellen zu erhöhen. Beispiele für solche Gruppierungen sind Zucker, wie Laktose, Maltose, Galaktose, sowie Polyethylenglycol.

Bevorzugt wird im Rahmen der vorliegenden Erfindung Polylysin als Polykation verwendet.

Gegebenenfalls ist das Polykation, oder eine Teilmenge davon, mit einem Internalisierungsfaktor für die Zielzellen konjugiert. Unter Internalisierungsfaktoren sind Liganden oder Fragmente davon zu verstehen, die nach Bindung an die Zelle über Endozytose, vorzugsweise Rezeptor-vermittelte Endozytose, internalisiert werden, oder Faktoren, deren Bindung/Internalisierung über Fusion mit Zellmembranelementen erfolgt. Beispiele für geeignete Internalisierungsfaktoren sind in der WO 93/07283 beschrieben. Die Verwendung eines Liganden ist vor allem dann vorteilhaft, wenn der zu transfizierende Zelltyp Rezeptoren exprimiert, die gut internalisieren, also "aktiv" sind.

Ein im Rahmen der vorliegenden Erfindung bevorzugt verwendeter Internalisierungsfaktor ist Transferrin.

Ob die Verwendung eines Internalisierungsfaktors erforderlich bzw. zweckmäßig ist, kann in Vergleichsversuchen auf einfache Weise ermittelt werden, indem untersucht wird, ob und in welchem Ausmaß die Gegenwart eines Internalisierungsfaktorkonjugats unter ansonsten gleichen Bedingungen eine Steigerung der Genexpression bewirkt.

Bevorzugt werden die Transfektionskomplexe hergestellt, indem die DNA zuerst mit einer Teilmenge an Polykation (und/oder Internalisierungsfaktor/Polykation-Konjugat) versetzt wird, wodurch eine gewisse Vorkondensation der DNA herbeigeführt werden dürfte, worauf die Hauptmenge an Polykation und/oder Internalisierungsfaktor/Polykation-Konjugat, z.B. Transferrin/Polylysin, beigegeben wird.

Vorzugsweise wird das Polykation, z.B. Polylysin, bzw. das Konjugat aus Polykation und Internalisierungsfaktor, z.B. Transferrin/Polylysin, oder eine Mischung davon, im Überschuß eingesetzt, so daß die DNA-Komplexe elektropositiv sind. Die im Rahmen der vorliegenden Erfindung durchgeführten Versuche haben gezeigt, daß, je nach Zelltyp, ein molarer Überschuß an positiver Ladung (z. B. in Form von Polylysin) von ca. 25% bis zum ca. zweifachen Überschuß gute Ergebnisse bringt.

Das erfindungsgemäße Verfahren ist besonders vorteilhaft bei der Anwendung auf Primärzellen, wie Fibroblasten.

Die Erfindung bezieht sich in einem weiteren Aspekt auf ein Verfahren zur Herstellung von Zellen, die ein heterologes Polypeptid von einem stabil transfizierten DNA-Molekül exprimieren. Dabei werden, im Anschluß an die Transfektion der Zellen in Gegenwart von Glycerin und/oder Ethylenglycol, gegebenenfalls mit einem Zusatz von lysosomatroper Substanz und/oder einem niederen Alkohol, mit einem Komplex aus einem für das heterologe Polypeptid kodierenden DNA-Molekül und einem Polykation, das gegebenenfalls ganz oder teilweise mit einem Internalisierungsfaktor konjugiert ist, die Zellen gezüchtet, stabile Zellklone selektioniert und daraus gegebenenfalls Zellinien etabliert.

Die Selektion stabiler Zellklone und die Etablierung von Zellinien werden nach herkömmlichen Methoden vorgenommen, solche Methoden können einschlägigen Handbüchern, z.B. Current Protocols in Molecular Biology, F.M. Ausubel et al. (Ed.), Vol. 1, Suppl. 14, 1987, 9.5, entnommen werden.

Diese Anwendung kommt z.B. für die Transfektion von eukaryotischen Zellen im Hinblick auf die Produktion rekombinanter Polypeptide oder im Hinblick auf die Herstellung von Tumorvakzinen in Betracht.

Die im Fall von Tumorzellen erhaltenen Zellinien, die von einem integrierten Gen ein immunstimulierendes Polypeptid, wie ein Zytokin, und/oder ein oder mehrere Tumorantigene exprimieren, sind für die Verwendung als Tumorvakzine geeignet.

Die Erfindung betrifft in einem weiteren Aspekt ein Transfektionsmedium, enthaltend als wirksame Komponente Komplexe aus Nukleinsäure und einem Polykation, das gegebenenfalls ganz oder teilweise mit einem Internalisierungsfaktor konjugiert ist, sowie Glycerin und/oder Ethylenglycol, sowie gegebenenfalls einen Zusatz an lysosomatroper Substanz und/oder einem niederen Alkohol. Neben den Transfektionskomponenten enthält das Medium die an den jeweiligen Zelltyp angepaßten üblichen Nährstoffe und Zusätze, z.B. in Form kommerziell erhältlicher Zellkulturmedien.

Im Rahmen der vorliegenden Erfindung wurde die Anwendung der vorliegenden Erfindung auf Zellen verschiedener Zellinien, auf primäre Zellen sowie zur Herstellung stabiler Zellklone, die ein heterologes Polypeptid exprimieren, gezeigt.

### Figurenübersicht

- Fig. 1:: Titration von Glycerin bei der Transfektion von humanen Melanomzellen
- Fig. 2:: Titration von Glycerin bei der Transfektion von humanen Melanomzellen
- Fig. 3:: Optimierung der Transfektionsbedingungen - Zeitverlauf
- Fig. 4:: Untersuchung der Effizienz verschiedener Gentransferkomplexe für die Transfektion von primären Melanomzellen in Gegenwart von Glycerin
- Fig. 5:: Titration der Ladung der Komplexkomponenten
- Fig. 6:: Ersatz von Transferrin-Polylysin durch Polylysin in positiv geladenen Komplexen
- Fig. 7:: Transfektion von primären humanen Fibroblasten mit einem Luciferase-Reporterplasmid in Gegenwart von Glycerin
- Fig. 8:: Transfektion von primären humanen Fibroblasten mit einem für IL-2 kodierenden Plasmid in Gegenwart von Glycerin
- Fig. 9:: Transfektion der Zellinie NIH 3T3 und einer Melanomzellinie in Gegenwart von Glycerin
- Fig. 10:: Transfektion von A549-Zellen in Gegenwart von 15 **%** Glycerin
- Fig. 11:: Transfektion von A549-Zellen in Gegenwart unterschiedlicher Glycerinmengen
- Fig. 12:: Transfektion von HeLa-Zellen und BNL CL.2-Zellen in Gegenwart von Glycerin
- Fig. 13:: Transfektion von primären humanen Melanomzellen in Gegenwart von Glycerin oder Threitol
- Fig. 14:: Untersuchung verschiedener Substanzen auf ihre Fähigkeit, die Gentransfereffizienz zu steigern
- Fig. 15:: Transfektion von primären Melanomzellen oder Fibroblasten in Gegenwart von Glycerin oder Ethylenglycol
- Fig. 16:: Transfektion von primären Fibroblasten in Gegenwart verschiedener mehrwertiger Alkohole oder DEAE-Dextran
- Fig. 17:: Herstellung stabiler Melanomzellklone

Die Ausführungsbeispiele illustrieren die Erfindung; es wurden, wenn nicht anders angegeben, die folgenden Materialien und Methoden verwendet (in einigen Beispielen wurde statt Polylysin streptavidinyliertes Polylysin verwendet; dieser Unterschied machte sich im Vergleich mit nicht-modifiziertem Polylysin bei der Effizienz der Genexpression nicht bemerkbar):

### i) Plasmid-DNA

Die Konstruktion des Plasmids pCMVL ist in der WO 93/07283, des Plasmids pGShIL2-tet in der WO 94/21808 beschrieben.

Das Plasmid pRSVneo wurde von Gorman, 1985, beschrieben.

### ii) Transferrin-Polylysin-Konjugate

Zur Synthese von Konjugaten aus Transferrin (Tf) und Polylysin (pL) mit einer Kettenlänge von 290 Lysinresten bzw. 250 Lysinresten (TfpL290 bzw. TfpL250) wurde die Wagner et al., 1991, beschriebene Methode eingesetzt. Das molare Verhältnis Transferrin/Polylysin betrug ca. 1:1.2.

### iii) Streptavidin-Polylysin-Konjugate (StpL)

Die Konjugate wurden hergestellt, wie in der WO 93/07283 beschrieben, wobei Polylysin 250 verwendet wurde.

### iv) Humane Melanomzellen H225

Primäre humane Melanomzellen wurden aus chirurgisch entfernten Melanomen isoliert. Die Tumore wurden mechanisch (mit Pinzette und chirurgischem Messer) in Gegenwart von RPMI 1640 Kulturmedium, enthaltend 5 % FCS, 2 mM Glutamin und Antibiotika, in kleine Fragmente zerteilt. Dann wurden die Gewebsfragmente vorsichtig mit dem Kolben einer Spritze durch ein Metallsieb gedrückt. Daraufhin wurde das Material mehrere Male durch Zentrifugation und Resuspension gewaschen, und die freigesetzten Zellen wurden in T25-Kulturflaschen ausgesät. Die Zellen wurden zu 100.000 Zellen pro Vertiefung transfiziert.

### v) Humanfibroblasten

Nach der chirurgischen Entfernung wurden Hautbiopsien in 4°C DMEM, enthaltend 10 % FCS, 2 mM Glutamin und Gentamycin, gegeben. Die Biopsien wurden in einer Gewebekultureinrichtung ausgiebig mit Pinzette und chirurgischem Messer im laminaren Luftstrom in sterilen 6 cm Plastikschalen zerkleinert. Dann wurden 3 ml DMEM, enthaltend 20 % FCS, 2 mM Glutamin und Antibiotika beigegeben, und die Kultur in einen 37°C Brutschrank gegeben. Nach 10 Tagen wurde das Medium durch DMEM, enthaltend 10 % FCS, ausgetauscht. Dann wurde das Medium weiter 2 x wöchentlich gewechselt. 4 Wochen nach Beginn der Kultur wurden die Zellen, die aus den Gewebsfragmenten herausgewachsen waren, trypsinisiert und für die Transfektion in neue Kulturschalen ausplattiert.

Eine alternative, bevorzugte Methode bestand darin, die Hautstücke nach der Zerkleinerung in frisches Medium zu überführen und nach Bedarf 1 bis 2 x mit Medium zu waschen. 5 bis 10 Gewebestücke wurden in eine T25-Gewebekulturflasche, deren Oberfläche mit DMEM plus 10 % FCS benetzt worden war, gegeben und gleichmäßig verteilt, worauf die Flasche umgedreht wurde. Dies bewirkte, daß die Biopsien herunterhängen ("hanging drop configuration"; diese Methode wurde von Jones, 1989, beschrieben). Nach 1 bis 3 h im Brutschrank wurden die Flaschen wieder umgedreht und mit 1 bis 2 ml Medium befüllt. Festsitzende Biopsien wurden nach 24 h auf 5 ml aufgefüllt; andernfalls wurde der Vorgang wiederholt. Nach 6 bis 10 Tagen wuchsen die ersten Fibroblasten aus, von diesem Zeitpunkt an wurde einmal wöchentlich das Medium gewechselt. Sobald die Zellen konfluent waren, wurden sie in eine T75-Flasche passagiert.

### vi) Zellinien

Die folgenden Zellinien stammen von ATCC: NIH 3T3 (ATCC CRL 1658), A549 (ATCC CCL 185) und TIB 73 (ATCC BNL CL.2).

### vii) Luciferase-Assay

Die Herstellung von Zellextrakten, die Standardisierung des Proteingehalts sowie die Bestimmung der Luciferaseaktivität wurden durchgeführt, wie in der WO 93/07283 beschrieben, wobei die Zellen in 250 mM TRIS pH 7.3, 0.5 % Triton X-100 geerntet und die Luciferaseaktivität von einem Aliquot des Überstandes gemessen wurde.

### viii) Interleukin-2-Assay

Die Expression von IL-2 wurde im Zellüberstand mit einem kommerziell erhältlichen ELISA (Bender MedSystems, Wien) bestimmt.

### ix) Chemikalien

Glycerin, Ethylenglycol, Diethylenglycol, Polyethylenglycol (PEG) 1000 und 6000 wurden von Fluka (Buchs, Schweiz), Threitol von Aldrich (Wien, Österreich) bezogen. (Die Konzentration von kommerziell erhältlichem Glycerin beträgt ca. 87 %. 10 % (v/v) bedeutet somit z.B. einen Zusatz von 200 µl des Ausgangspräparats zu einem Endvolumen des Transfektionsmediums von 2 ml.)

### Beispiel 1

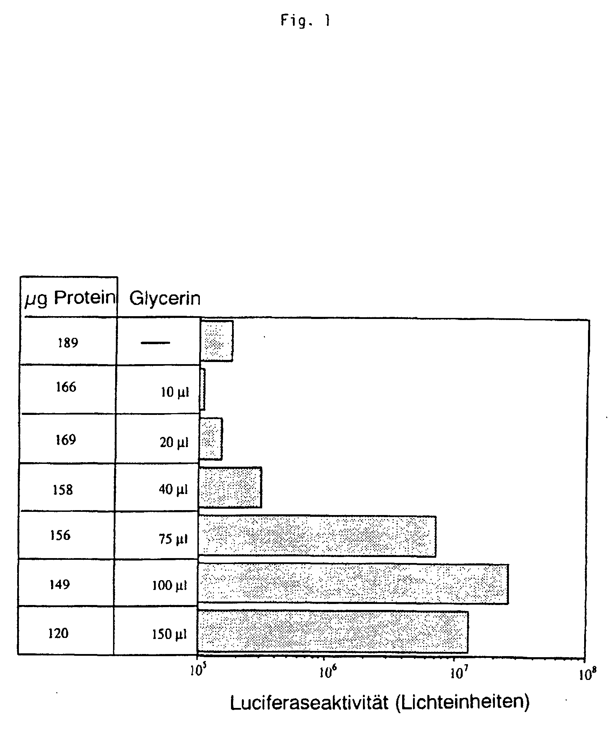

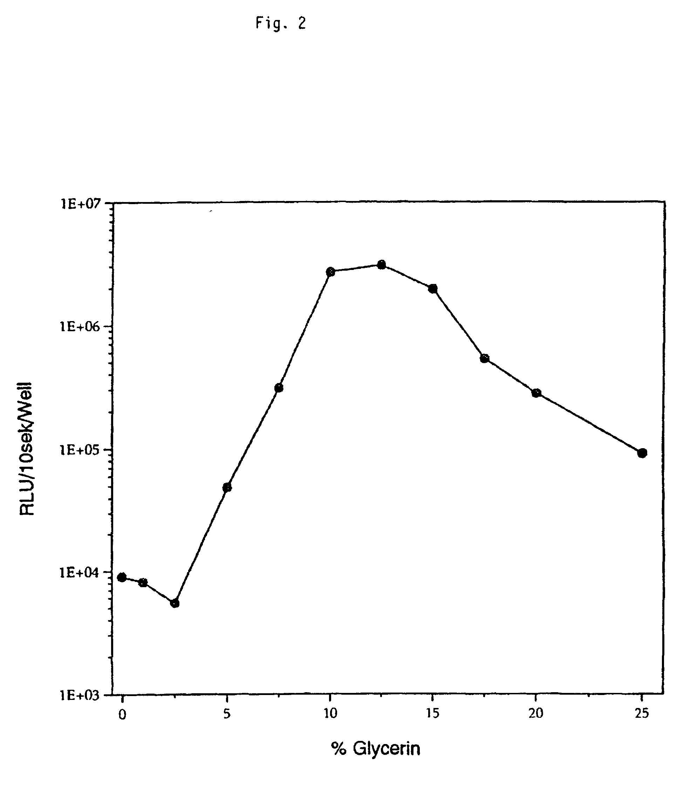

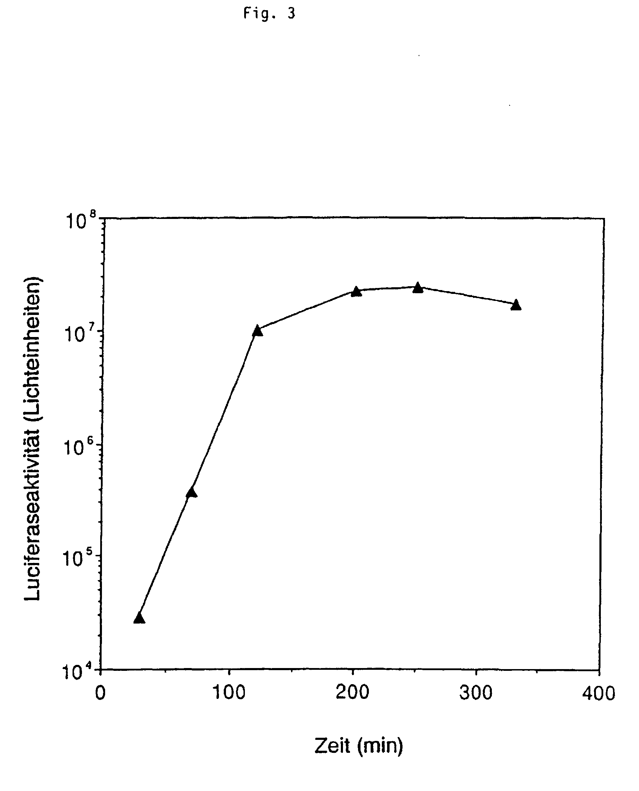

Titration von Glycerin bei der Transfektion von humanen Melanomzellen
a) 0.4 µg Streptavidin-modifiziertes Polylysin wurden in 75 µl HBS verdünnt und nach Zugabe von 3 µg pCMVL in 75 µl HBS 30 min inkubiert. Anschließend wurden 3 µg Transferrin-Polylysin (TfpL290) in 75 µl HBS zugegeben und weitere 30 min inkubiert. Nach Zugabe der jeweils in Fig. 1 angegebenen Menge Glycerin wurden 700 µl Medium (RPMI 1640 plus FCS, 2 mM L-Glu, 1 mM Natriumpyruvat, Antibiotika) zugegeben und das Gemisch auf die Zellen pipettiert. Das Transfektionsmedium wurde nach 4 h abgesaugt und die Zellen mit frischem Medium (ohne Transfektionskomponenten) versetzt. Das Ernten der Zellen und die Messung der Luciferaseaktivität erfolgte nach 24 h nach Standardprotokoll. Die Ergebnisse der Transfektionen sind in Fig. 1 dargestellt (die nach Bradford bestimmte Proteinmenge des Zellysates ist angegeben). Steigende Mengen an Glycerin im Medium bewirkten bis zu einer Konzentration von 10 % (1.15 M) eine Steigerung der Luciferaseaktivität. Bei einem Glyceringehalt von 15 % wurde ein Abfall der Expression beobachtet.
b) Ähnlich wie in a) wurden 100.000 H225-Zellen pro Vertiefung mit Komplexen, bestehend aus 1.5 µg pCMVL, 0.2 µg pL und 1.5 µg TfpL in Gegenwart von Glycerin in den in Fig. 2 angegebenen Konzentrationen (v/v) transfiziert. Es zeigte sich, daß 10 % Glycerin (1.15 M) die besten Ergebnisse brachte; eine Konzentration von unter 4 % bewirkte keine Erhöhung der Reportergenexpression, während Konzentrationen ab 15 % für die H225-Zellen toxisch waren. Die Luciferasewerte sind die Durchschnittswerte von Doppelbestimmungen und stellen relative Lichteinheiten pro mg Protein und 10 sek dar.
c) Um die Transfektionsbedingungen zu optimieren, wurde ein Zeitverlaufsversuch durchgeführt, wobei die Transfektionen wie in b) in Gegenwart von 10 % (v/v) Glycerin vorgenommen wurden. Nach den in Fig. 3 angegebenen Zeitpunkten wurde das Medium ersetzt. Es zeigte sich, daß die höchsten Expressionswerte erhalten wurden, wenn die Zellen 3 bis 4 h lang mit den Komplexen und Glycerin inkubiert wurden.

### Beispiel 2

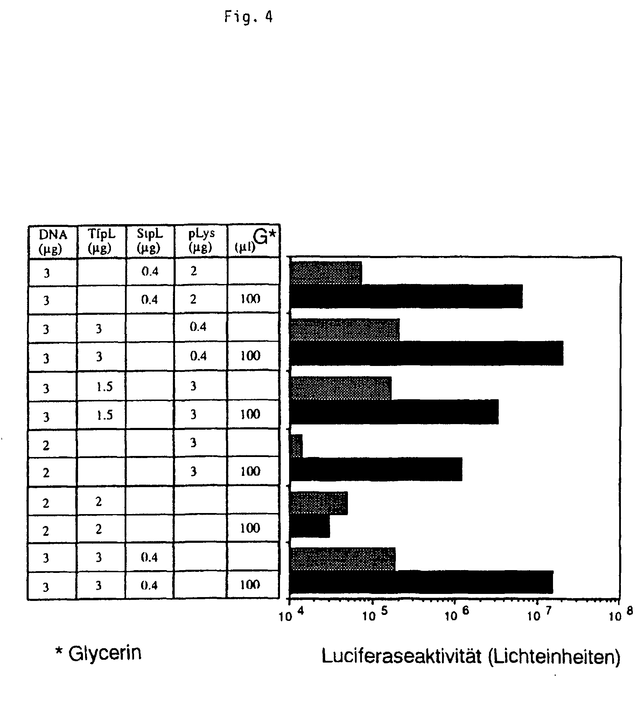

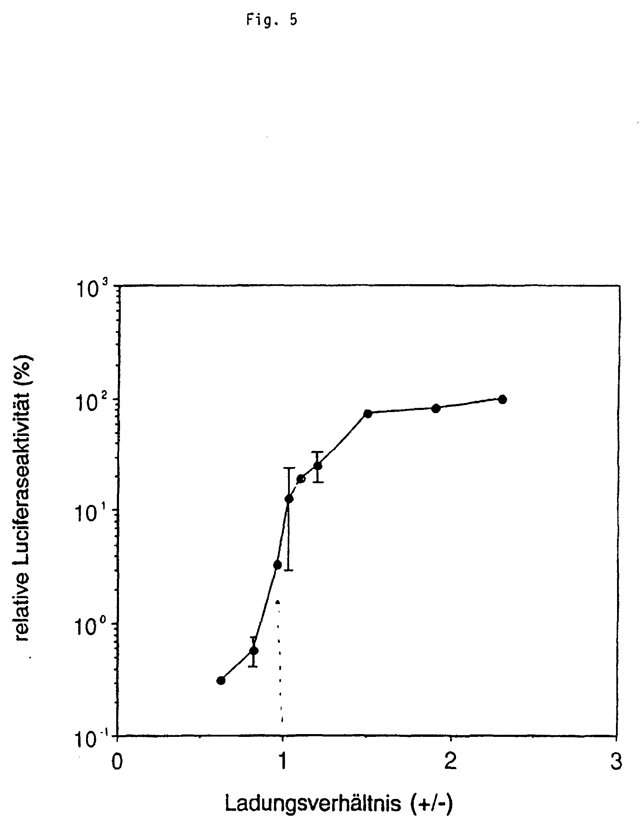

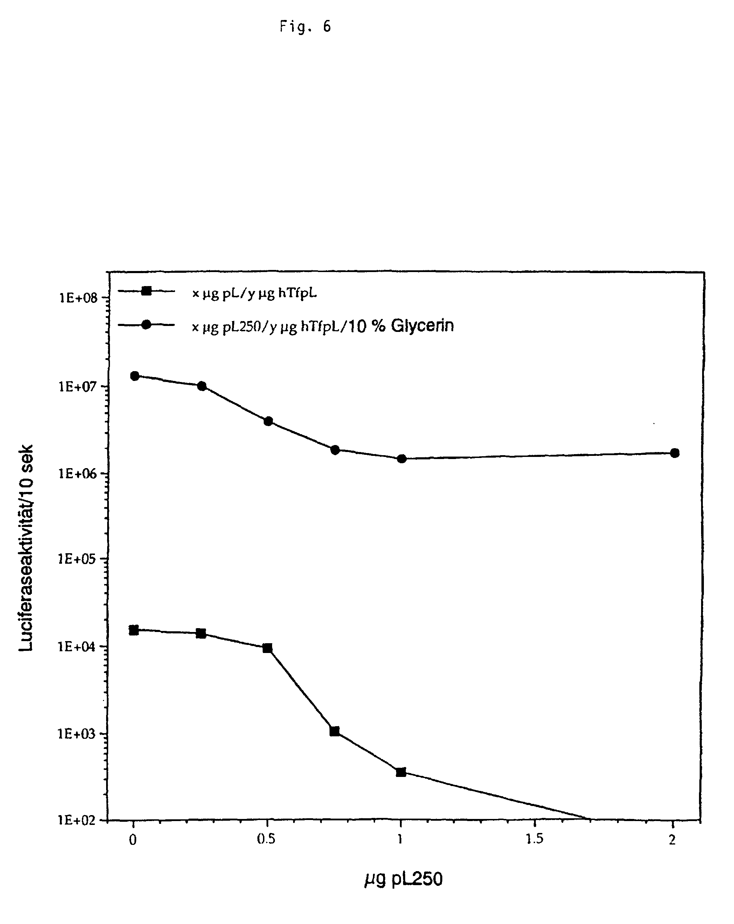

Untersuchung der Effizienz verschiedener Gentransferkomplexe für die Transfektion von primären Melanomzellen in Gegenwart von Glycerin
a) 0.4 µl StpL oder pL300 in 75 µl HBS wurden mit 3 µg pCMVL in 75 µl HBS für 30 min inkubiert, dann wurden 3 pg TfpL290 oder pL in 75 µl HBS zugegeben und weitere 30 min inkubiert. DNA/pL- und DNA/TfpL-Komplexe wurden durch Mischen der Komponenten in 75 µl HBS und 30minütige Inkubation hergestellt. Elektropositive DNA/TfpL/pL-Komplexe wurden durch 30minütige Inkubation von 3 µg DNA und 1.5 pg TfpL290 und anschließender Zugabe von 3 µg pL hergestellt. Nach Zugabe von Glycerin und Medium wurden die Komplexe für 4 h auf die primären Melanomzellen der Bezeichnung H225 gegeben, anschließend wurde das Medium durch frisches ersetzt. Das Ernten der Zellen und die Messung der Luciferaseaktivität erfolgte nach 24 h. Die Ergebnisse der Transfektionen sind in Fig. 4 dargestellt: Die Zellen, die in Gegenwart von 10 % Glycerin (die Konzentrationsangaben beziehen sich in diesem und den folgenden Beispielen auf das Gesamtvolumen) transfiziert wurden, zeigten bis auf eine Ausnahme eine deutlich höhere Expression als ohne Glycerin.
b) In einem weiteren Experiment zur Titration der Ladung wurden H225-Zellen, wie in den vorigen Beispielen angegeben, mit Komplexen aus 1.5 µg pCMVL (4.5 nmol negative Ladungen), 0.2 µg pL und steigenden Mengen TfpL transfiziert. In Fig. 5 sind die relativen Expressionswerte in Abhängigkeit des Ladungsverhältnisses (mol positive Ladungen/mol negative Ladungen) dargestellt. Es zeigte sich, daß die Effizienz des Gentransfers mit der Gesamtladung des Komplexes korreliert; ein zumindest geringer Überschuß an positiven Ladungen erwies sich für einen effizienten Gentransfer als erforderlich.
c) H225-Zellen wurden in Gegenwart oder Abwesenheit von 10 % Glycerin mit Komplexen, bestehend aus 1.5 µg DNA und mit Mischungen von TfpL/pL, die ein Ladungsverhältnis (plus/minus) von 1.5 ergeben, transfiziert. Das Ergebnis ist in Fig. 6 dargestellt; es zeigt, daß TfpL in positiv geladenen Komplexen durch pL ersetzt werden kann, ohne die Reportergenexpression wesentlich herabzusetzen.

### Beispiel 3

### Transfektion von primären humanen Fibroblasten in Gegenwart von Glycerin

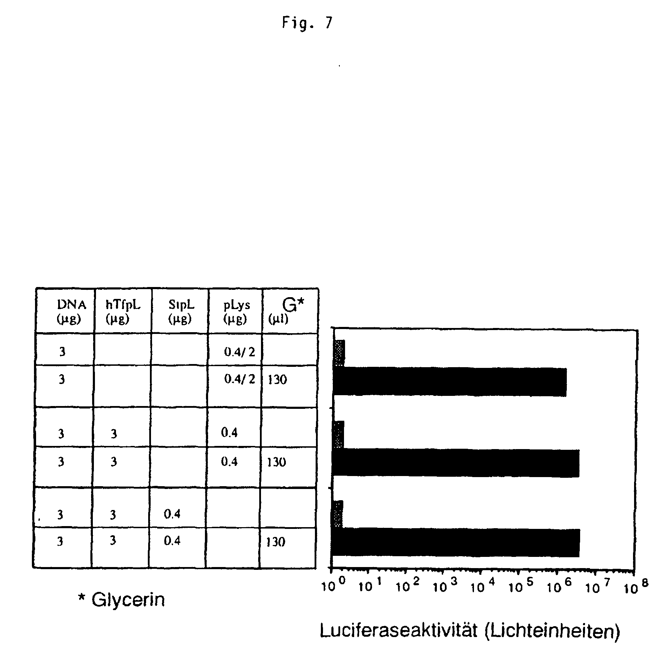

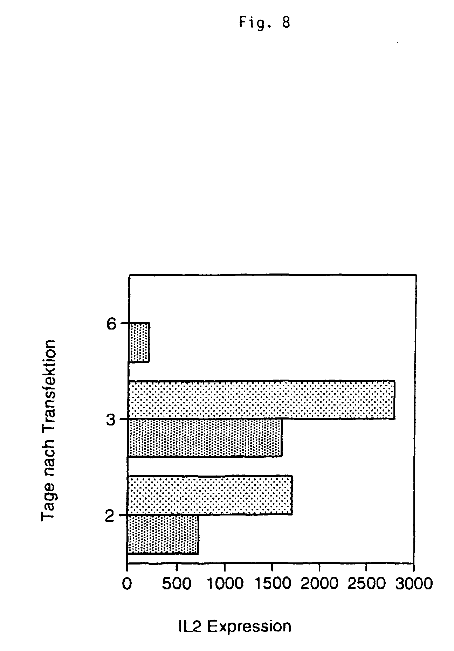

a) Transfektion mit einem Luciferase-Reporterplasmid
   0.4 µg pL bzw. StpL in 75 µl HBS wurden mit 3 µg pCMVL in 75 µl HBS für 30 min inkubiert, dann wurden 3 µg TfpL290 oder 2 µg pL in 75 µl HBS zugegeben und weitere 30 min inkubiert. Nach Zugabe von Medium und 13 % Glycerin zu den Komplexen wurde das Transfektionsmedium für 4 h auf die Zellen gegeben (45.000 Zellen pro Vertiefung). Das Ernten der Zellen und die Messung der Luciferaseaktivität erfolgte nach 24 h. Die Ergebnisse der Transfektionen sind in Fig. 7 dargestellt.
b) Transfektion mit einem für IL-2 kodierenden Plasmid
   0.9 µg Polylysin in 75 µl HBS wurden mit 6 µg pCMVL in 75 µl HBS für 30 min inkubiert, dann wurden 6 µg TfpL250 in 75 µl HBS zugegeben und weitere 30 min inkubiert. Nach Zugabe von Medium und 10 bzw. 15 % Glycerin wurden die Transfektionskomplexe für 4 h auf die Zellen gegeben (200.000 Zellen/6 cm Kulturschale). Das Medium wurde im Anschluß daran durch frisches Medium ersetzt und in der Folge täglich gewechselt sowie die IL-2 -Expression jeweils im Überstand mittels ELISA nach Vorschrift des Herstellers gemessen. Die Ergebnisse der Transfektionen sind in Fig. 8 dargestellt, wobei die dunkleren Balken die Expression in Gegenwart von 15 % Glycerin und die helleren Balken die Expression in Gegenwart von 10 % Glycerin zeigen.

### Beispiel 4

### Transfektion von Zellinien in Gegenwart von Glycerin

### a) NIH 3T3-Zellen

### Zellen einer Melanomzellinie

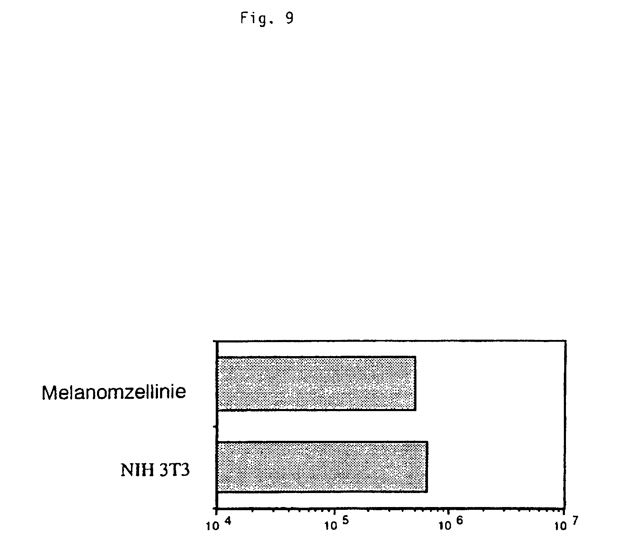

Die Transfektionskomplexe wurden aus 3 µg pCMVL, 3 µg TfpL und 0.4 µg StpL hergestellt, wie in den vorangegangenen Beispielen beschrieben. Nach Zugabe von 75 µl Glycerin wurde mit DMEM-Medium auf 1 ml ergänzt. Das Transfektionsmedium wurde auf je 50.000 Zellen der Zellinie NIH 3T3 (ATCC CRL 1658) oder einer Zellinie, erhalten aus Melanomzellen, die einer hohen Zahl von Passagen unterworfen worden waren, aufgegeben (jeweils 50.000 Zellen pro Vertiefung). Nach 4 h wurde das Transfektionsmedium durch frisches Medium ergänzt, wie in den vorangegangenen Beispielen beschrieben. Die Ergebnisse der Transfektionen sind in Fig. 9 dargestellt.

### b) Lungenkarzinomzellen (Zellinie A549)

### i) Transfektion in Gegenwart von 15 % Glycerin

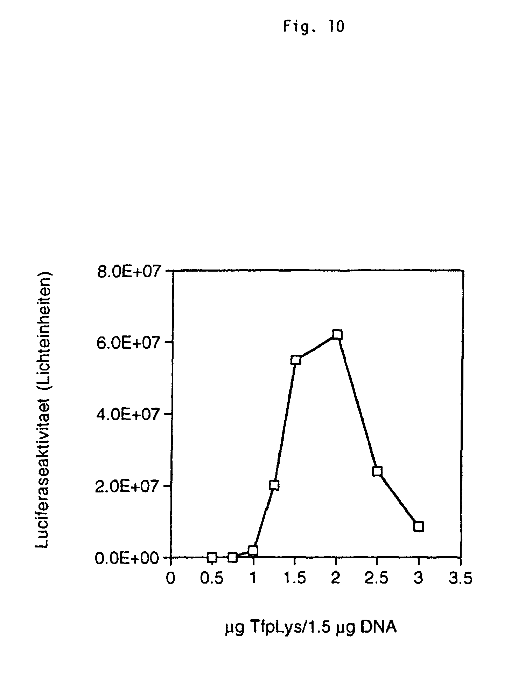

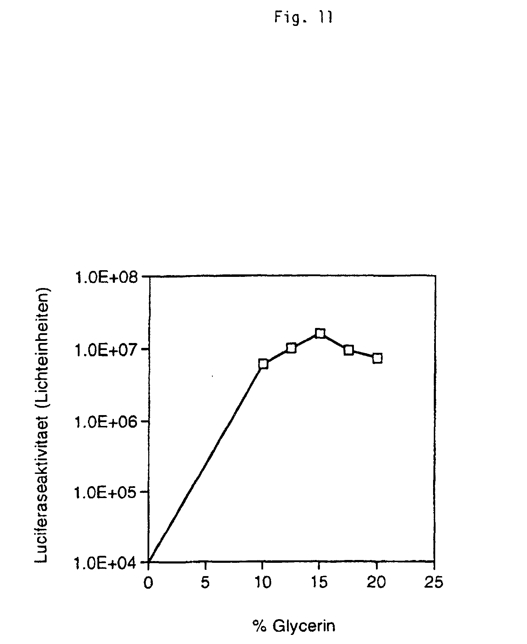

Die Transfektionskomplexe wurden aus 1.5 µg pCMVL-DNA und den in Fig. 10 angegebenen Mengen TfpL250 hergestellt, wie in den vorigen Beispielen angegeben. Nach Zugabe von Medium (DMEM/10 % FCS) und Glycerin (Endkonzentration 15 %) wurden die Komplexe für 4 h auf je 100.000 Zellen pro Vertiefung einer 6-Lochplatte aufgegeben. Dann wurde das Medium gewechselt und nach 24 h die Luciferaseaktivität gemessen. Das Ergebnis der Versuche ist in Fig. 10 dargestellt.

### ii) Transfektion in Gegenwart unterschiedlicher Mengen an Glycerin

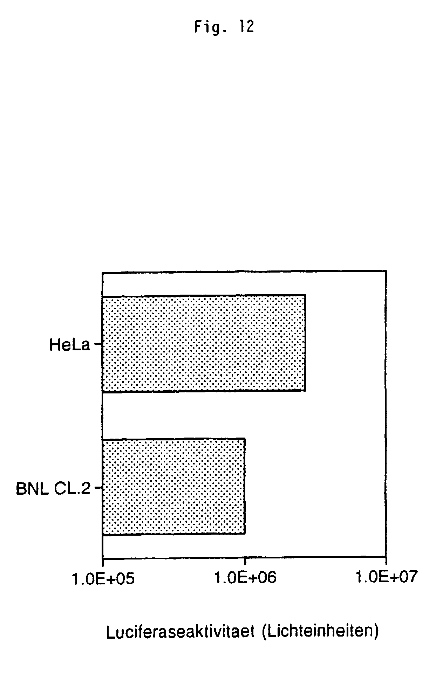

Es wurde nach dem in i) beschriebenen Versuchsschema vorgegangen, wobei die Transfektionskomplexe aus 1.5 µg pCMVL-DNA und 1.5 µg TfpL250 hergestellt wurden. Nach Zugabe von Medium und unterschiedlichen Mengen Glycerin in den in Fig. 11 angegebenen Konzentrationen wurden die Zellen behandelt, wie unter i) angegeben, und die Luciferaseaktivität gemessen (Fig. 11).

### c) HeLa-Zellen

### BNL CL.2-Zellen

Die Transfektionskomplexe wurden aus 1.5 pg pCMVL-DNA und 1.5 µg TfpL290 hergestellt, wie in den vorangegangenen Beispielen beschrieben. Für die Transfektion der HeLa-Zellen wurde DMEM-Medium mit 10 % Glycerin, für die Transfektion der BNL CL.2-Zellen High Glucose DMEM-Medium (plus 10 % FCS, Glutamin, Antibiotika) mit 15 % Glycerin eingesetzt. Nach 4 h wurde jeweils wieder durch frisches Medium ergänzt und nach 24 h die Luciferaseaktivität gemessen; die Ergebnisse sind in Fig. 12 dargestellt.

### Beispiel 5

### Untersuchung des Einflusses anderer Substanzen als Ersatz von Glycerin

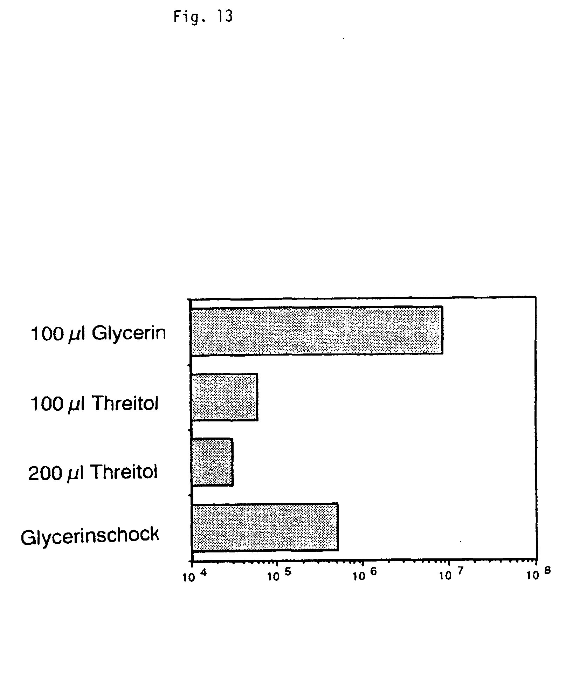

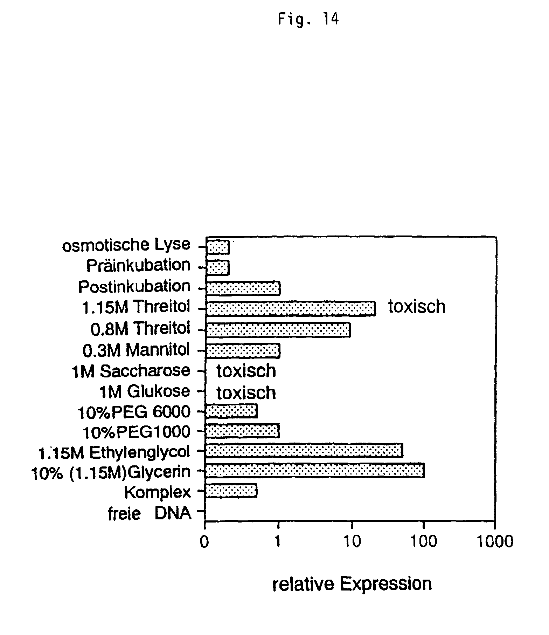

a) Transfektion von primären humanen Melanomzellen in Gegenwart von Glycerin oder Threitol
   Die Transfektionskomplexe in diesem Beispiel wurden hergestellt, wie in Beispiel 2 beschrieben. Vor der Transfektion wurden dem Medium 10 % Glycerin oder 10 % bzw. 20 % Threitol (50 %) beigegeben. Die Transfektionen wurden pro 100.000 Zellen durchgeführt, wie in Beispiel 2 beschrieben.
   In einem Parallelversuch wurde nach der vierstündigen Inkubation mit den Transfektionskomplexen und dem Absaugen der Komplexe Glycerin in einer Konzentration von 20 % für 15 min auf die Zellen aufgegeben, um einen osmotischen Schock herbeizuführen, dann wurde das Glycerin abgesaugt. Die Ergebnisse der Transfektionen sind in Fig. 13 dargestellt: Die Behandlung mit Threitol statt mit Glycerin während der Transfektion brachte wesentlich schlechtere Expressionswerte als mit Glycerin. Außerdem zeigte sich, daß die Schockbehandlung mit Glycerin wesentlich weniger effizient ist als die Gegenwart von Glycerin während der gesamten Dauer der Transfektion.
b) In diesem Experiment wurden verschiedene Substanzen, die Glycerin chemisch ähnlich sind, auf ihre Fähigkeit, die Gentransfereffizienz zu steigern, untersucht. Dazu wurden H225-Zellen mit Komplexen, bestehend aus 3 µg pCMVL, 0.4 µg pL und 3 µg TfpL in Gegenwart der in Fig. 14 angegebenen Substanzen in den angegebenen Konzentrationen transfiziert. Die in der obersten Spalte dargestellte osmotische Lyse wurde nach der von Okada und Rechsteiner, 1982, beschriebenen Methode mit den DNA/TfpL-Komplexen, gemischt in hypertonischem Medium (Dauer: 3 h bzw. 20 min; Medium/0.5 M Glucose/10 % PEG 1000), durchgeführt. Im Anschluß an die Transfektion wurden die Zellen 2 mal mit hypotonischem Medium (Medium/Wasser = 6/4) gewaschen und 3 min hypotonisch gehalten, bevor sie in normalem Medium weiterkultiviert wurden (die unterschiedliche Behandlungsdauer in hypertonischem Medium brachte identische Ergebnisse, es ist daher nur ein Wert in der Fig. dargestellt; entsprechende Versuche mit BNL CL.2-Zellen brachten ähnliche Ergebnisse). Keine der verwendeten Substanzen außer Ethylenglycol, das bei gleicher Molarität unter den untersuchten Bedingungen 30 bis 50 % der Wirksamkeit von Glycerin zeigte, erwies sich als geeignet, die Reportergenexpression wesentlich zu steigern. Die geringere Aktivität von Ethylenglycol war zumindest teilweise auf eine toxische Wirkung zurückzuführen. Alle anderen Substanzen waren bei einer Konzentration deutlich unter 1.15 M (entspricht für Glycerin 10 %) toxisch. Die in den Spalten 2 und 3 dargestellten Werte wurden erhalten, indem die Zellen vor bzw. nach der Transfektion mit 10 % Glycerin inkubiert wurden; sie zeigen, daß der Effekt von Glycerin von dessen Gegenwart während der Transfektion abhängt; weder die Vor- noch die Nachbehandlung (in der Figur "Prä-" bzw. "Postinkubation") mit Glycerin verstärkten die Reportergenexpression wesentlich.

### Beispiel 6

### Transfektion von primären Melanomzellen oder Fibroblasten in Gegenwart von Glycerin oder Ethylenglycol

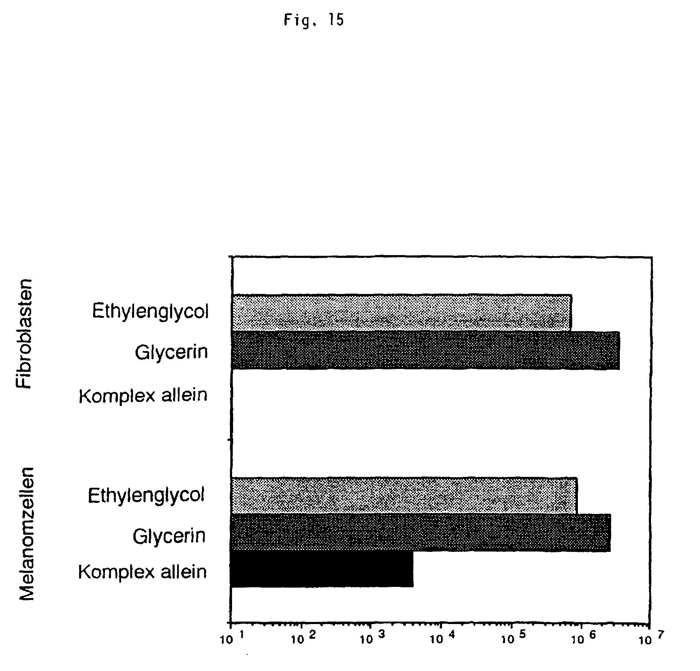

Die Transfektionskomplexe wurden hergestellt, wie in den vorigen Beispielen beschrieben. Dem Transfektionsmedium wurde vor der Aufbringung auf die Zellen 10 % Glycerin bzw. Ethylenglycol beigegeben. Pro Transfektion wurden 100.000 Melanomzellen bzw. 50.000 Fibroblasten eingesetzt. Die Ergebnisse der Transfektionen sind in Fig. 15 dargestellt: Bei den Fibroblasten konnte der Komplex ohne Zusatz keine Expression bewirken; bei Melanomzellen brachte der Zusatz von Ethylenglycol oder Glycerin eine Steigerung um etwa drei Zehnerpotenzen. Die absoluten Expressionswerte bei Zusatz der mehrwertigen Alkohole waren bei beiden Zelltypen etwa gleich.

### Beispiel 7

### Transfektion von primären Fibroblasten in Gegenwart verschiedener mehrwertiger Alkohole

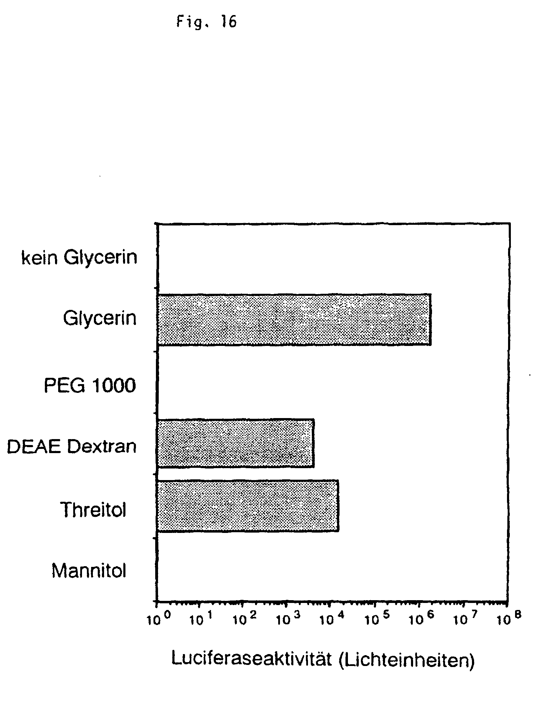

Die Transfektionskomplexe in diesem Beispiel wurden hergestellt, wie in Beispiel 3 beschrieben. Vor der Transfektion wurden dem Medium 13 % Glycerin, 20 % Polyethylenglycol 1.000 (50 %), 250 µg/ml DEAE-Dextran oder Mannitol (170 mM) oder 20 % Threitol (50 %) beigegeben; eine Probe wurde ohne irgend einen Zusatz transfiziert. Die Transfektionen wurden pro 40.000 Zellen durchgeführt, wie in Beispiel 3 beschrieben. Die Ergebnisse der Transfektionen sind in Fig. 16 dargestellt: Es zeigte sich, daß ohne Glycerinzugabe bzw. bei Zugabe von Polyethylenglycol oder Mannitol keine Expression erfolgte; die Zugabe von Threitol brachte eine gegenüber der DEAE-Dextran-Methode nur geringfügig verbesserte Expression. Auch dieses Ergebnis weist darauf hin, daß die Wirkung von Glycerin nicht osmotisch ist; wäre dies der Fall, müßten auch die höherwertigen Alkohole eine ähnliche Wirkung zeigen.

### Beispiel 8

### Herstellung stabiler Melanomzellklone

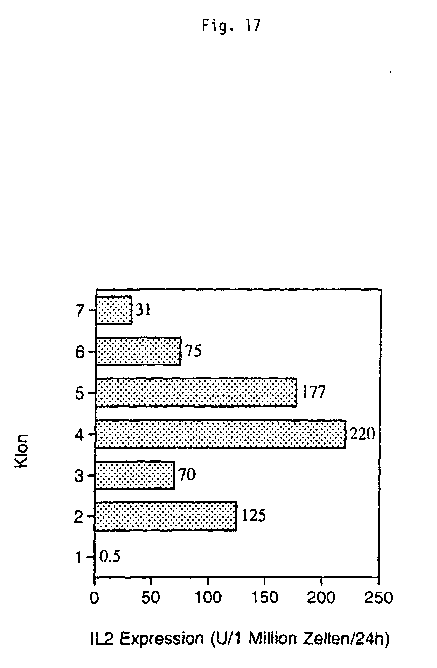

Für die Herstellung der Transfektionskomplexe wurde zunächst das mit XmnI linearisierte Plasmid pGShIL2-tet mit XmnI-linearisiertem Plasmid pRSVneo im Verhältnis 10:1 (6 µg pGShIL2-tet/0.6 pg pRSVneo) in 100 µl HBS gemischt. 7.5 µg TfpL250 in 100 µl HBS wurden zugegeben und 30 min bei Raumtemperatur inkubiert. Die Mischung wurde mit Medium (RPMI 1640, 1 mM Natriumpyruvat), enthaltend 10 % Glycerin, auf H225-Zellen (300.000/T25-Kulturflasche; Hersteller: Nunc) gegeben. Nach 4 h wurde durch frisches Medium ersetzt. Nach 60 h wurde die Selektion mittels Medium, enthaltend 3 mg/ml Geneticin (G418, Sigma), begonnen, nach 5 Tagen wurde die Konzentration von G418 auf 1 mg/ml verringert. Die Zellen wurden in Medium, enthaltend 1 mg/ml G418, passagiert. Für die Bestimmung der IL-2-Expression wurden 400.000 Zellen pro Vertiefung in eine 6-Lochplatte plattiert. Nach 24 h wurde das Medium durch 2 ml frisches Medium ersetzt. Nach weiteren 24 h Inkubation wurde das im Überstand enthaltene IL-2 mittels ELISA nach Vorschrift des Herstellers gemessen. Die Zellzahl wurde mittels Zählung nach Trypsinierung bestimmt. Die in Fig. 17 angegebenen Werte entsprechen IL-2-Einheiten pro 24 h pro 10⁶ Zellen.

### Beispiel 9

### Untersuchung des Einflusses weiterer Substanzen und Parameter auf ihre Fähigkeit, eine Steigerung der durch Glycerin hervorgerufenen Gentransfereffizienz zu bewirken

In diesen Versuchen, deren Ergebnisse in der Tabelle dargestellt sind, wurden die Transfektionen für den jeweiligen Zelltyp durchgeführt, wie in den vorigen Beispielen beschrieben; die verwendeten Transfektionskomplexe wiesen die folgende Zusammensetzung auf:
A: 1.5 µg DNA/0.2 µg pL/1.25 µg TfpL
B: : 1.5 µg DNA/1.5 µg TfpL
C: 1.5 µg DNA/0.2 µg pL/1 µg pL (pL wurde in 2 Portionen zugegeben)
D: 1.5 µg DNA/1.2 µg pL

Chloroquin wurde in einer Konzentration von 100 µM, Bafilomycin Al in einer Konzentration von 200 nM, Ethanol in einer Konzentration von 1 % bzw. 1.5 % eingesetzt. Das Gesamtvolumen des Transfektionsmediums betrug jeweils 1 ml.

Die Ergebnisse dieser Versuche sind in der Tabelle dargestellt; die angegebenen Expressionswerte sind auf die relativen Lichteinheiten bezogen, die mit Komplex A in Gegenwart von 10 % Glycerin erhalten wurden (= 100 %). 100 % entspricht 1.5 x 10⁷ Lichteinheiten pro 10⁵ H225-Zellen, 1.5 x 10⁶ Lichteinheiten pro 10⁵ NIH 3T3-Zellen, 10⁷ Lichteinheiten pro 10⁵ A549-Zellen oder 1.2 x 10⁶ Lichteinheiten pro 10⁵ primäre humane Fibroblasten.

In einem weiteren Experiment (nicht in der Tabelle enthalten) wurde die Konzentration des Komplexes erhöht, indem das Volumen des Transfektionsmediums von 1 ml auf 700 µl verringert wurde (die Glycerinkonzentration betrug 10 %, die Ethanolkonzentration 1.5 %). Diese Maßnahme ergab eine Steigerung der Luciferaseexpression auf das Fünffache.

**Tabelle**

| Zellen | Komplex | Glycerin % (v/v) | relative Luciferase-expression (%) | Substanz |
|---|---|---|---|---|
| H225 | A | 0 | 0.7 | - |
| | A | 0 | 0.3 | 1 % Ethanol |
| | A | 0 | 4 | Chloroquin |
| | A | 0 | 0.8 | Bafilomycin A1 |
| | A | 10 | 100 | - |
| | A | 10 | 200 | 1 % Ethanol |
| | A | 10 | 200 | 1.5 % Ethanol |
| | A | 10 | 300 | Chloroquin |
| | A | 10 | 240 | Bafilomycin A1 |
| | B | 10 | 200 | 1.5 % Ethanol |
| | B | 10 | 50 | - |
| | C | 10 | 95 | - |
| | D | 10 | 70 | - |
| | | | | |
| NIH 3T3 | A | 0 | 0 | - |
| | A | 12.5 | 100 | - |
| | B | 0 | 0 | - |
| | B | 0 | 0 | Bafilomycin A1 |
| | B | 0 | 35 | Chloroquin |
| | B | 12.5 | 200 | - |
| | B | 12.5 | 350 | Bafilomycin A1 |
| | B | 12.5 | 80 | Chloroquin |
| | C | 0 | 0 | - |
| | C | 12.5 | 260 | - |
| | D | 0 | 0 | - |
| | D | 12.5 | 1000 | - |
| | B | 10 | 200 | - |
| | B | 10 | 1000 | Bafilomycin A1 |
| A549 | A | 0 | 0 | - |
| | A | 15 | 100 | - |
| | B | 0 | 0 | - |
| | B | 15 | 150 | - |
| | B | 15 | 170 | Chloroquin |
| | C | 15 | 130 | - |
| | D | 15 | 60 | - |
| | | | | |
| primäre Fibroblasten | 4xA | 0 | 0 | - |
| | 4xA | 13 | 100 | - |
| | 4xC | 0 | 0 | - |
| | 4xC | 13 | 50 | - |

### Literatur

Bowman, E.J., Siebers, A. und Altendorf, K., 1988, Proc.Natl.Acad.Sci. USA 85, 7972-7976.
Chen, C.A. und Okayama, H., 1988, BioTechniques 6, 632-638.
Cotten, M., Wagner, E., Zatloukal, K., Phillips, S., Curiel, D. und Birnstiel, M.L., 1992, Proc.Natl.Acad.Sci. USA 89, 6094-6098.
Curiel, D.T., Agarwal, S., Wagner, E. und Cotten, M., 1991, Proc.Natl.Acad.Sci. USA 88, 8850-8854.
Curiel, D.T., Agarwal, S., Romer, M.U., Wagner, E., Cotten, M., Birnstiel, M.L. und Boucher, R.C., 1992a, Am.J.Respir.Cell and Mol.Biol. 6, 247-252.
Curiel, D.T., Wagner, E., Cotten, M., Birnstiel, M.L., Agarwal, S., Li, Ch.-M., Loechel, S. und Hu, P.-H., 1992b, Human Gene Therapie 3, 147-154.
Gorman, C., 1985, DNA Cloning, A practical approach, Vol. 2, D.M. Glover (Ed.), 143-190.
Kasid, A., Morecki, S., Aebersold, P., Cornetta, K., Culver, K., Freeman, S., Director, E., Lotze, M.T., Blaese, R.M., Anderson, W.F. und Rosenberg, S.A., 1990, Proc.Natl.Acad.Sci. USA 87, 473-477.
Okada, C.Y. und Rechsteiner, M., 1982, Cell 29, 33-41.
Parker, B.A. und Stark, G.R., 1979, J. Virology 31, 360-369.
Seglen, P.O., 1983, Methods Enzymol. 96, 737-764.
Wagner, E., Zenke, M., Cotten, M., Beug, H. und Birnstiel, M.L., 1990, Proc.Natl.Acad.Sci. USA 87, 3410-3414.
Wagner, E., Cotten, M., Mechtler, K., Kirlappos, H. und Birnstiel, M.L., 1991, Bioconjugate Chemistry 2, 226-231.
Wagner, E., Zatloukal, K., Cotten, M., Kirlappos, H., Mechtler, K., Curiel, D.T. und Birnstiel, M.L., 1992, Proc.Natl.Acad.Sci. USA 89, 6099-6103.
Wilson, J.M., Danos, O., Grossman, M., Raulet, D.H. und Mulligan, R.C., 1990, Proc.Natl.Acad.Sci. USA 87, 439-443.
Wu, G.Y. und Wu, C.H., 1987, J. Biol. Chem. 262, 4429-4432.
Yoshimori, T., Yamamoto, A., Moriyama, Y., Futai, M. und Tashiro, Y., 1991, J. Biol. Chem. 266, 17707-17712.
Zatloukal, K., Wagner, E., Cotten, M., Phillips, S., Plank, C., Steinlein, P., Curiel, D. und Birnstiel, M.L., 1992, Ann.New York Acad.Sci. 660, 136-153.

## Patentansprüche

1. Verfahren zum Einbringen von Nukleinsäure/Polykation-Komplexen in höhere eukaryotische Zellen, **dadurch gekennzeichnet, daß** man die Komplexe in Gegenwart von Ethylenglycol und/oder Glycerin auf die Zellen aufbringt.

2. Verfahren nach Anspruch 1, **dadurch gekennzeichnet, daß** man als Nukleinsäure/Polykation-Komplexe DNA/Polylysin-Komplexe einsetzt.

3. Verfahren nach Anspruch 1 oder 2, daß das Polykation ganz oder teilweise mit einem Internalisierungsfaktor für die Zellen konjugiert ist.

4. Verfahren nach Anspruch 3, **dadurch gekennzeichnet, daß** der Internalisierungsfaktor Transferrin ist.

5. Verfahren nach einem der vorhergehenden Ansprüche, **dadurch gekennzeichnet, daß** man das Polykation und/oder das Internalisierungsfaktor/Polykation-Konjugat im Überschuß einsetzt, so daß die DNA-Polykation Komplexe elektropositiv sind.

6. Verfahren nach einem der vorhergehenden Ansprüche, **dadurch gekennzeichnet, daß** man die DNA zuerst mit einer Teilmenge an, gegebenenfalls konjugiertem, Polykation versetzt, worauf man die Hauptmenge an Polykation und/oder Internalisierungsfaktor/Polykation-Konjugat zugibt.

7. Verfahren nach einem der vorhergehenden Ansprüche, **dadurch gekennzeichnet, daß** man Glycerin in einer Konzentration von ca. 8 bis ca. 15 %, insbesondere ca. 8 bis ca. 13 %, bezogen auf das Gesamtvolumen des Transfektionsmediums, einsetzt.

8. Verfahren nach einem der vorhergehenden Ansprüche, **dadurch gekennzeichnet, daß** man als Zellen Primärzellen verwendet.

9. Verfahren nach Anspruch 8, **dadurch gekennzeichnet, daß** man Fibroblasten verwendet.

10. Verfahren zur Herstellung von Zellen, die ein heterologes Polypeptid von einem stabil transfizierten DNA-Molekül exprimieren, **dadurch gekennzeichnet, daß** man Zellen in Gegenwart von Glycerin und/oder Ethylenglycol mit einem Komplex aus einem für das heterologe Polypeptid kodierenden DNA-Molekül und einem Polykation, das gegebenenfalls ganz oder teilweise mit einem Internalisierungsfaktor konjugiert ist, transfiziert, die Zellen züchtet, stabile Zellklone selektioniert und gegebenenfalls aus den erhaltenen Klonen Zellinien etabliert.

11. Verfahren nach Anspruch 10, **dadurch gekennzeichnet, daß** man Glycerin in einer Konzentration von ca. 8 bis ca. 15 %, insbesondere ca. 8 bis ca. 13 %, bezogen auf das Gesamtvolumen des Transfektionsmediums, einsetzt.

12. Verfahren nach einem der vorhergehenden Ansprüche, **dadurch gekennzeichnet, daß** man außerdem eine Substanz, die die Ansäuerung der Endosomen verhindert, und/oder einen niederen Alkohol zusetzt.

13. Verfahren nach Anspruch 12, **dadurch gekennzeichnet, daß** man Chloroquin zusetzt.

14. Verfahren nach Anspruch 12, **dadurch gekennzeichnet, daß** man Bafilomycin zusetzt.

15. Verfahren nach Anspruch 12, **dadurch gekennzeichnet, daß** man Ethanol zusetzt.

16. Verfahren nach einem der Ansprüche 10 bis 15, **dadurch gekennzeichnet, daß** die Zellen Tumorzellen sind.

17. Verfahren nach Anspruch 16, **dadurch gekennzeichnet, daß** das heterologe Polypeptid ein immunstimulierendes Polypeptid, insbesondere ein Zytokin ist.

18. Verfahren nach Anspruch 17, **dadurch gekennzeichnet, daß** die Zellen Melanomzellen sind und das Zytokin Interleukin-2 ist.

19. Medium für die Transfektion von höheren eukaryotischen Zellen, enthaltend neben Nährstoffen und üblichen Zusätzen als wirksame Komponente Komplexe aus Nukleinsäure und einem, gegebenenfalls ganz oder teilweise mit einem Internalisierungsfaktor für die Zellen konjugierten, Polykation, **dadurch gekennzeichnet, daß** es Ethylenglycol und/oder Glycerin enthält.

20. Transfektionsmedium nach Anspruch 19, **dadurch gekennzeichnet, daß** es ca. 8 bis ca. 15 %, insbesondere ca. 8 bis ca. 13 %, bezogen auf das Gesamtvolumen, Glycerin enthält.

21. Transfektionsmedium nach Anspruch 19 oder 20, **dadurch gekennzeichnet, daß** es außerdem eine Substanz, die die Ansäuerung der Endosomen verhindert, und/oder einen niederen Alkohol enthält.

22. Transfektionsmedium nach Anspruch 21, **dadurch gekennzeichnet, daß** es Chloroquin enthält.

23. Transfektionsmedium nach Anspruch 21, **dadurch gekennzeichnet, daß** es Bafilomycin enthält.

24. Transfektionsmedium nach Anspruch 21, **dadurch gekennzeichnet, daß** es Ethanol enthält.

## Claims

1. Process for introducing nucleic acid/polycation complexes into higher eukaryotic cells, **characterised in that** the complexes are applied to the cells in the presence of ethyleneglycol and/or glycerol.

2. Process according to claim 1, **characterised in that** DNA/polylysine complexes are used as the nucleic acid/polycation complexes.

3. Process according to claim 1 or 2, **characterised in that** the polycation is wholly or partially conjugated with an internalising factor for the cells.

4. Process according to claim 3, **characterised in that** the internalising factor is transferrin.

5. Process according to one of the preceding claims, **characterised in that** the polycation and/or the internalising factor/polycation conjugate is used in excess, so that the DNA-polycation complexes are electropositive.

6. Process according to one of the preceding claims, **characterised in that** the DNA is first mixed with a partial amount of optionally conjugated polycation, after which the majority of polycation and/or internalising factor/polycation conjugate is added.

7. Process according to one of the preceding claims, **characterised in that** glycerol is used in a concentration of about 8 to about 15%, particularly about 8 to about 13%, based on the total volume of the transfection medium.

8. Process according to one of the preceding claims, **characterised in that** primary cells are used as the cells.

9. Process according to claim 8, **characterised in that** fibroblasts are used.

10. Process for preparing cells which express a heterologous polypeptide from a stably transfected DNA molecule, **characterised in that** cells are transfected in the presence of glycerol and/or ethyleneglycol with a complex of a DNA molecule coding for the heterologous polypeptide and a polycation which is optionally wholly or partially conjugated with an internalising factor, the cells are cultivated, stable cell clones are selected and, optionally, cell lines are established from the clones obtained.

11. Process according to claim 10, **characterised in that** glycerol is used in a concentration of about 8 to about 15%, more particularly about 8 to about 13%, based on the total volume of the transfection medium.

12. Process according to one of the preceding claims, **characterised in that** additionally a substance which prevents the acidification of the endosomes and/or a lower alcohol is added.

13. Process according to claim 12, **characterised in that** chloroquine is added.

14. Process according to claim 12, **characterised in that** bafilomycin is added.

15. Process according to claim 12, **characterised in that** ethanol is added.

16. Process according to one of claims 10 to 15, **characterised in that** the cells are tumour cells.

17. Process according to claim 16, **characterised in that** the heterologous polypeptide is an immuno-stimulant polypeptide, more particularly a cytokine.

18. Process according to claim 17, **characterised in that** the cells are melanoma cells and the cytokine is interleukin-2.

19. Medium for the transfection of higher eukaryotic cells, containing in addition to nutrients and conventional additives, as the active components, complexes of nucleic acid and a polycation which is optionally fully or partially conjugated with an internalising factor for the cells, **characterised in that** it contains ethyleneglyclol and/or glycerol.

20. Transfection medium according to claim 19, **characterised in that** it contains about 8 to about 15%, more particularly about 8 to about 13% of glycerol, based on the total volume.

21. Transfection medium according to claim 19 or 20, **characterised in that** it also contains a substance which prevents the acidification of the endosomes, and/or a lower alcohol.

22. Transfection medium according to claim 21, **characterised in that** it contains chloroquine.

23. Transfection medium according to claim 21, **characterised in that** it contains bafilomycin.

24. Transfection medium according to claim 21, **characterised in that** it contains ethanol.

## Revendications

1. Procédé pour introduire des complexes acide nucléique/polycation dans des cellules eucaryotes supérieures **caractérisé en ce que** l'on applique les complexes aux cellules en présence d'éthylèneglycol et/ou de glycérine.

2. Procédé selon la revendication 1 **caractérisé en ce que** l'on utilise des complexes ADN/polylysine comme complexes acide nucléique/polycation.

3. Procédé selon la revendication 1 ou 2 **caractérisé en ce que** le polycation est conjugué totalement ou partiellement avec un facteur d'internalisation pour les cellules.

4. Procédé selon la revendication 3 **caractérisé en ce que** le facteur d'internalisation est la transferrine.

5. Procédé selon l'une des revendications précédentes **caractérisé en ce que** l'on utilise le polycation et/ou le conjugué facteur d'internalisation/polycation en excès de sorte que les complexes ADN-polycation sont électropositifs.

6. Procédé selon l'une des revendications précédentes **caractérisé en ce que** l'on ajoute à l' ADN tout d'abord une quantité partielle de polycation éventuellement conjugué, après quoi on ajoute la quantité principale de polycation et/ou de conjugué facteur d'internalisation/polycation.

7. Procédé selon l'une des revendications précédentes **caractérisé en ce que** l'on utilise la glycérine en une concentration d'environ 8 à environ 15 %, en particulier d'environ 8 à environ 13 %, par rapport au volume total du milieu de transfection.

8. Procédé selon l'une des revendications précédentes **caractérisé en ce que** l'on utilise des cellules primaires comme cellules.

9. Procédé selon la revendication 8 **caractérisé en ce que** l'on utilise des fibroblastes.

10. Procédé de production de cellules qui expriment un polypeptide hétérologue à partir d'une molécule d'ADN transfectée de manière stable, **caractérisé en ce que** l'on transfecte des cellules en présence de glycérine et/ou d'éthylèneglycol avec un complexe constitué par une molécule d'ADN codant le polypeptide hétérologue et par un polycation, qui est éventuellement conjugué totalement ou partiellement avec un facteur d'internalisation, on cultive les cellules, on sélectionne des clones cellulaires stables et, éventuellement, on établit des lignées cellulaires à partir des clones obtenus.

11. Procédé selon la revendication 10 **caractérisé en ce que** l'on utilise la glycérine en une concentration d'environ 8 à environ 15 %, en particulier d'environ 8 à environ 13 %, par rapport au volume total du milieu de transfection.

12. Procédé selon l'une des revendications précédentes **caractérisé en ce que** l'on ajoute en outre une substance qui empêche l'acidification des endosomes, et/ou un alcool inférieur.

13. Procédé selon la revendication 12 **caractérisé en ce que** l'on ajoute de la chloroquine.

14. Procédé selon la revendication 12 **caractérisé en ce que** l'on ajoute de la bafilomycine.

15. Procédé selon la revendication 12 **caractérisé en ce que** l'on ajoute de l'éthanol.

16. Procédé selon l'une des revendications 10 à 15 **caractérisé en ce que** les cellules sont des cellules tumorales.

17. Procédé selon la revendication 16 **caractérisé en ce que** le polypeptide hétérologue est un polypeptide immunostimulant, en particulier une cytokine.

18. Procédé selon la revendication 17 **caractérisé en ce que** les cellules sont des cellules de mélanome et la cytokine est l'interleukine-2.

19. Milieu pour la transfection de cellules eucaryotes supérieures contenant, outre des substances nutritives et des additifs habituels, comme composant efficace des complexes d'acide nucléique et d'un polycation éventuellement conjugué totalement ou partiellement avec un facteur d'internalisation pour les cellules, **caractérisé en ce qu'**il contient de l'éthylèneglycol et/ou de la glycérine.

20. Milieu de transfection selon la revendication 19 **caractérisé en ce qu'**il contient environ 8 à environ 15 %, en particulier environ 8 à environ 13 %, par rapport au volume total, de glycérine.

21. Milieu de transfection selon la revendication 19 ou 20 **caractérisé en ce qu'**il contient en outre une substance qui empêche l'acidification des endosomes, et/ou un alcool inférieur.

22. Milieu de transfection selon la revendication 21 **caractérisé en ce qu'**il contient de la chloroquine.

23. Milieu de transfection selon la revendication 21 **caractérisé en ce qu'**il contient de la bafilomycine.

24. Milieu de transfection selon la revendication 21 **caractérisé en ce qu'**il contient de l'éthanol.
